(19) Europäisches Patentamt
European Patent Office
Office européen des brevets

(11) **EP 2 031 402 B1**

(12) **EUROPEAN PATENT SPECIFICATION**

(45) Date of publication and mention
of the grant of the patent:
**30.05.2012 Bulletin 2012/22**

(51) Int Cl.:
***G01N 33/86*** (2006.01)

(21) Application number: **08162829.9**

(22) Date of filing: **22.08.2008**

(54) **Method for measuring coagulation time of blood sample**

Verfahren zum Messen der Koagulationszeit einer Blutprobe

Procédé pour la mesure du temps de coagulation d'un échantillon de sang

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **31.08.2007 JP 2007226463**

(43) Date of publication of application:
**04.03.2009 Bulletin 2009/10**

(73) Proprietor: **SYSMEX CORPORATION
Kobe-shi,
Hyogo 651-0073 (JP)**

(72) Inventors:
• **Hoshiko, Susumu
1chome
Chuo-ku
Kobe-shi Hyogo 651-0073 (JP)**
• **Kobayashi, Katsushi
1 chome
Chuo-ku
Kobe-shi Hyogo 651-0073 (JP)**

(74) Representative: **De Clercq, Ann G. Y. et al
De Clercq & Partners cvba
Edgard Gevaertdreef 10 a
9830 Sint-Martens-Latem (BE)**

(56) References cited:
**WO-A1-2004/062689 US-A- 5 091 304
US-A- 5 591 403 US-A- 6 100 072**

• **"Stability Constants (log K1) of Various Metal Chelates"[Online] 26 October 2006 (2006-10-26), pages 1-8, XP002504903 Retrieved from the Internet: URL:http://george-eby-research.com/html/st ability_constants.html>**
• **TOH CHENG HOCK ET AL: "Biphasic transmittance waveform in the APTT coagulation assay is due to the formation of a Ca++-dependent complex of C-reactive protein with very-low-density lipoprotein and is a novel marker of impending disseminated intravascular coagulation" BLOOD, AMERICAN SOCIETY OF HEMATOLOGY, US, vol. 100, no. 7, 1 October 2002 (2002-10-01), pages 2522-2529, XP002376810 ISSN: 0006-4971**

**Description**

TECHNICAL FIELD

The present invention relates to use of a reagent and a method for measuring coagulation time of a blood sample.

BACKGROUND

Blood coagulation is the reaction in which various blood coagulation factors in blood are involved. By this reaction, fibrinogen in blood is converted finally into fibrin. The time required for blood coagulation (hereinafter referred to sometimes as blood coagulation time) can be measured to examine e.g. shortages and abnormalities of various blood coagulation factors. Based on the blood coagulation time, the bleeding tendency of a patient can also be examined.

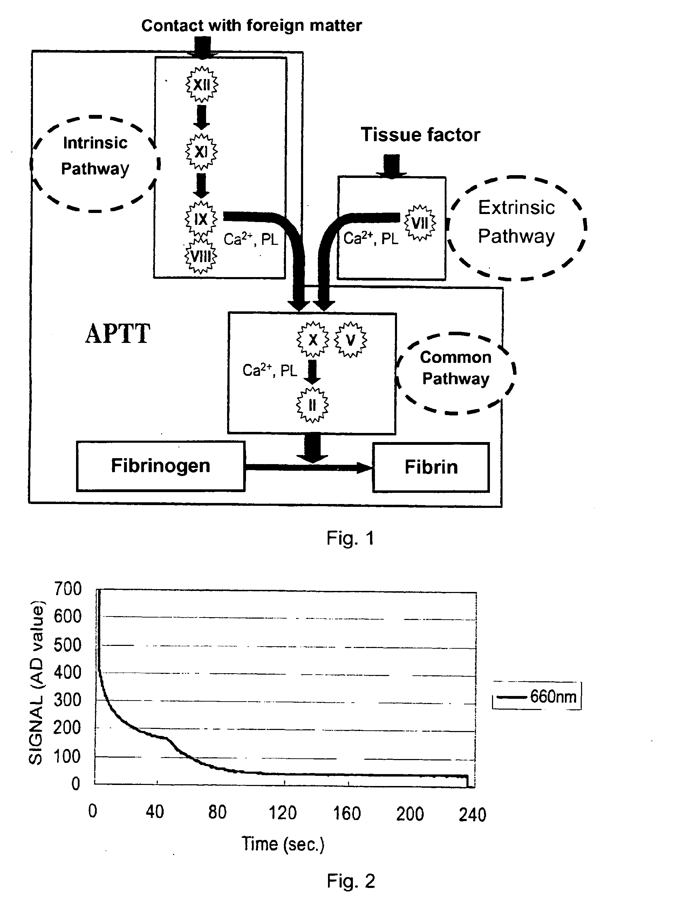

Usually, blood coagulation is estimated to occur through the following pathways (see FIG. 1). That is, the first pathway is a pathway called extrinsic (tissue factor) coagulation. Starting from the release of tissue thromboplastin (also called a tissue factor) by epidermic cells or the like, the coagulation factor VII is activated by the tissue thromboplastin. This activated coagulation factor VII activates the coagulation factor X. This activates the coagulation factor V and factor II, and finally fibrinogen is converted into fibrin. The blood coagulation time in this pathway can be measured by a prothrombin time (PT) measurement method.

The second pathway is a pathway called intrinsic (contact factor) coagulation. In the intrinsic coagulation pathway, the coagulation factor XII is activated for example by contact with a negatively charged surface of a foreign matter such as collagen. In turn, the activated factor XII activates the factor XI. The activated factor XI in turn activates the factor IX, and the activated factor IX activates the factor X with collaborative action of calcium ions and the factor VIII. Then, the factor V and the factor II are sequentially activated, and finally fibrinogen is converted into fibrin. The blood coagulation time in this pathway can be measured by an activated partial thromboplastin time (APTT) method.

Calcium and phospholipid (PL) are involved in both the pathways.

Known methods of measuring blood coagulation time include an optical measurement method which comprises mixing a blood sample containing blood coagulation factors with a reagent containing phospholipid (PL) and calcium to cause blood coagulation and detecting formation of fibrin as an optical change. The PT measurement method or the APTT measurement method is one kind of optical measurement method.

In such an optical measurement method, an optical change is usually very low after a reagent for measuring blood coagulation is added to a blood sample to initiate the blood coagulation reaction until the reaction proceeds to form fibrin. Then, a rapid optical change occurs within a short time after formation of fibrin.

However, calcium contained in a reagent for measuring blood coagulation may sometimes react with a substance other than blood coagulation factors contained in a blood sample to generate agglutination. The substance other than blood coagulation factors which reacts with calcium includes, for example, C reactive protein (CRP) and lipid components (for example, very low density lipoprotein (VLDL)) contained in a blood sample. When agglutination is generated by such reaction, the agglutination is detected as an optical change at an initial stage of measurement, and the optical change may sometimes be recognized erroneously as blood coagulation (Toh, CH et al., Blood, 100(7), pp. 2522-2529 (2002)).

US2003/138962 discloses a method of detecting an abnormality in an early reaction in optical measurement of blood coagulation by setting at least one checkpoint or check region from the initiation of measurement to the end of the coagulation reaction.

However, there has not been a reagent capable of accurately measuring the blood coagulation time by suppressing the generation of an optical change at an initial stage of the measurement as described above.

SUMMARY

The reagent for measuring coagulation time comprises a calcium chelator. This calcium chelator can interfere with the reaction between calcium and a substance other than a coagulation factor in the blood sample, while this substantially does not interfere with coagulation reaction.

The reagent kit for measuring coagulation time comprises a first and second reagent. The first reagent comprises at least one selected from the group consisting of partial thromboplastin, tissue thromboplastin, and complex of tissue factor and phospholipid. The second reagent comprises calcium. In the first reagent, the second reagent or both, a calcium chelator is included. This calcium chelator can interfere with the reaction between calcium and substance other than a coagulation factor in the blood sample, while this substantially does not interfere with coagulation reaction.

The method for measuring coagulation time according to the present invention comprises a contacting step and a measuring step. The contacting step is to contact the blood sample, calcium, and a compound selected from the group consisting of partial thromboplastin, tissue thromboplastin, and complex of tissue factor and phospholipid, in the presence of a calcium chelator. This calcium chelator can interfere with the reaction between calcium and substance other than a coagulation factor in the blood sample, while this substantially does not interfere with the coagulation reaction. The measuring step is to measure the coagulation time of the blood sample.

Use of the reagent for measuring blood coagulation and the method for measuring blood coagulation time according to the present invention can be used to measure blood coagulation time by suppressing generation of an optical change at an initial stage of the measurement. Accordingly, blood coagulation can be measured more accurately than by conventional methods of measuring blood coagulation time.

BRIEF DESCRIPTION OF THE DRAWINGS

Fig. 1 is a diagram which shows two main pathways of blood coagulation.
Fig. 2 is a graph which shows the results of a reference experiment 1.
Fig. 3 is a graph which shows the results of a reference experiment 2.
Fig. 4 is a graph which shows the results of experiment 1.

DESCRIPTION OF THE PREFERRED EMBODIMENTS

The blood sample used in the measurement of blood coagulation is prepared usually by adding an anticoagulant such as sodium citrate to blood (whole blood or plasma) collected from a patient. Because the anticoagulant contained in the blood sample influences the reaction in measurement of blood coagulation, the influence of the anticoagulant is eliminated by allowing a large amount of calcium to be usually present in the blood coagulation measurement system to bind the calcium to the anticoagulant, thereby interfering with the action of the anticoagulant.

Calcium is also an essential component for allowing the blood coagulation reaction to proceed as shown in FIG. 1. That is, calcium present excessively in the blood coagulation measurement system, together with phospholipid (PL) allowed to be present in the blood coagulation measurement system, reacts with blood coagulation factors in the blood sample thereby activating them so that the blood coagulation reaction proceeds.

An excess of calcium that does not react with the anticoagulant or is not used in the blood coagulation reaction agglutinates by reacting with substances in the blood sample such as lipid particles of very low density lipoprotein (VLDL) and C reactive protein (CRP) or other than blood coagulation factors. This agglutination causes an optical change at an initial stage of the measurement. In the optical measurement, the optical change caused by this agglutination and the optical change by the blood coagulation reaction are hardly distinguished from each other as described above. It follows that when the agglutination as described above occurs, there is a possibility that the blood coagulation time cannot be accurately measured.

The present inventors paid notice to calcium as one of the causative substances generating an optical change at an initial stage of measurement. Then, the inventors thought that an optical change at an initial stage of the measurement can be suppressed by chelating calcium with a chelator to prevent calcium from reacting with other substances (that is, substances other than blood coagulation factors in a blood sample) causing an optical change at an initial stage of the measurement. However, calcium has a role in reacting with an anticoagulant to interfere with the action of the anticoagulant and has a role in reacting with blood coagulation factors in a blood sample to advance the blood coagulation reaction, as described above. Therefore, complete chelating of calcium with a chelator could lead to failure of calcium to react with an anticoagulant or with blood coagulation factors in a blood sample. Consequently, the inventors found that when a suitable calcium chelator having a weaker power to bind to calcium than the anticoagulant and blood coagulation factors is selected and used, an optical change at an initial stage of the measurement can be suppressed to measure the blood coagulation time, and the present invention was thereby completed.

The reagent for measuring blood coagulation is a reagent used for measurement of blood coagulation time, and contains a calcium chelator. This calcium chelator interferes with the reaction between calcium and substances other than coagulation factors in a blood sample, but does not substantially interfere with the blood coagulation reaction. The reagent for measuring blood coagulation may further contain other components such as calcium and phospholipid used in measurement of blood coagulation time so as to be usable in measurement of blood coagulation by mixing the reagent alone with a blood sample. The reagent for measuring blood coagulation may be a reagent kit usable in measurement of blood coagulation, which is combined with a plurality of reagents containing other components used in the measurement of blood coagulation time.

Various methods are known for measuring blood coagulation. The reagent for measuring blood coagulation can be used particularly preferably in an optical blood coagulation measurement method of detecting the blood coagulation reaction by measuring optical information such as a change in transmitted light and a change in scattered light. The optical blood coagulation measurement method includes, for example, prothrombin time (PT) measurement method, an activated partial thromboplastin time (APTT) measurement method, a lupus anticoagulant (LA) measurement method, a thrombotest, a hepaplastintest, and a coagulation factor quantification method.

For example, the APTT measurement method known in the art is a method using phospholipid (also called partial thromboplastin), an APTT reagent containing an activator, and a calcium solution. Specifically, the APTT reagent is mixed with a blood sample (plasma), and the resulting mixture is incubated. After incubation, the mixture is mixed with the calcium solution to measure coagulation time. The activator used in the APTT measurement method includes ellagic acid, kaolin, celite etc.

The reagent for measuring blood coagulation, when used in the APTT measurement method for example, may be an independent reagent other than the APTT reagent or the calcium solution, may be a reagent containing the chelator together with phospholipid (that is, a reagent having the chelator added to the APTT reagent known in the art), or may be a reagent containing both the chelator and calcium.

The PT measurement method is a method using a tissue factor/phospholipid complex or tissue thromboplastin and a PT reagent containing calcium. Specifically, this method involves mixing the PT reagent with a blood sample (plasma) to measure the coagulation time.

The reagent for measuring blood coagulation, when used in the PT measurement method for example, may be an independent reagent other than the PT reagent or may be a reagent containing the chelator together with phospholipid and calcium (that is, a reagent having the chelator added to the PT reagent known in the art). In the PT measurement method, a tissue factor/phospholipid complex or tissue thromboplastin, and calcium, may be separate reagents. In this case, the reagent for measuring blood coagulation may be a reagent containing both the chelator and phospholipid (that is, a reagent having the chelator added to a reagent containing a tissue factor/phospholipid complex or tissue thromboplastin) or may be a reagent containing both the chelator and calcium.

The LA measurement method is a method of detecting an anti-phospholipid antibody in a blood sample by a coagulation test. Various methods for measuring LA are known and include, for example, a method of using two reagents different in the concentration of phospholipid. Specifically, the LA measurement method is a method that involves measuring the blood coagulation time of a blood sample by using two reagents different in the concentration of phospholipid and then using a difference or ratio between the determined 2 blood coagulation times to detect an anti-phospholipid antibody in the blood sample. In the LA measurement method, a method of measuring coagulation time varies depending on the type of a reagent. For example, when the reagent used in the LA measurement method contains calcium and an activator, the coagulation time is measured based on the principle of APTT measurement. When the reagent used in the LA measurement contains calcium and a snake venom, the coagulation time is measured based on the principle of snake venom measurement time. When the reagent used in the LA measurement contains calcium and a tissue factor/ phospholipid complex or tissue thromboplastin, the coagulation time is measured based on the principle of PT measurement.

The thrombotest is one of the methods of detecting an abnormality in the activity of vitamin K-dependent coagulation factor. The thrombotest is a method wherein a reagent containing tissue thromboplastin derived from bovine brain, bovine plasma adsorbed on barium sulfate, and calcium are added to whole blood or plasma as a sample to measure coagulation time. In the thrombotest, calcium and another composition may be separate reagents.

The hepaplastintest is a method of detecting an abnormality in the activity of vitamin K-dependent coagulation factor. The hepaplastintest is used to measure coagulation time in essentially the same manner as in the thrombotest except that tissue thromboplastin derived from rabbit brain is used in place of tissue thromboplastin derived from bovine brain.

The coagulation factor quantification method is a method of quantifying individual coagulation factors by using the APTT measurement method or PT measurement method. Specifically, this method involves adding plasma deficient in only an objective coagulation factor to plasma as a sample and measuring coagulation time by using the measurement principle of the APTT measurement method or PT measurement method. When the objective coagulation factor is a coagulation factor participating in intrinsic coagulation, the reagent used in the APTT measurement method is used. When the objective coagulation factor is a coagulation factor participating in extrinsic coagulation or in common coagulation, the reagent used in the PT measurement method is used.

The reagent for measuring blood coagulation may be a reagent containing the calcium chelator together with components contained in a conventionally known reagent used in each of the measurement methods or may be a calcium chelator-containing independent reagent used in combination with a conventionally known reagent used in each of the measurement methods.

The chelator used in the reagent for measuring blood coagulation is preferably that which has a lower chelate stability constant for calcium than that of the anticoagulant contained in a blood sample. Specifically, the chelator is preferably that which has a lower chelate stability constant for calcium than that of citric acid or its salt used generally as an anticoagulant. The chelator having such chelate stability constant has a calcium-binding ability weaker than that of the anticoagulant in a blood sample or than that of the blood coagulation factor.

In this specification, "chelate stability constant for calcium" is a value calculated as log $K_{MA}$ determined by a method described in a table on pp. 5 to 10 in an appendix to a reference "Kinzoku Chelate (Metal Chelate) III, First Edition, ed. Takeichi Sakaguchi & Keihei Ueno."

A higher chelate stability constant for calcium is indicative of easier chelating with calcium.

The chelate stability constant of citric acid for calcium is 3.16 according to the reference "Kinzoku Chelate (Metal Chelate) III, First Edition, ed. Takeichi Sakaguchi & Keihei Ueno."

Specifically, the chelate stability constant of the chelator for calcium is preferably 3 or less, more preferably 2.5 or less.

Examples of the chelator having a chelate stability constant of 3 or less for calcium include organic acids such as maleic acid, malic acid, malonic acid, lactic acid, formic acid, acetic acid, butyric acid, isobutyric acid, valeric acid, isovaleric acid, succinic acid, fumaric acid, citraconic acid, itaconic acid, tricarballylic acid, propane-1,1,2,3-tetracarboxylic acid, butane-1,2,3,4-tetracarboxylic acid, glycolic acid, thioglycolic acid, tartaric acid, isocitric acid, pyruvic acid and oxaloacetic acid, and salts thereof. These chelators may be used singly or in a combination of two or more thereof.

The chelator is preferably at least one member selected from the group consisting of maleic acid, malic acid, malonic acid, lactic acid, and salts thereof. According to the reference "Kinzoku Chelate (Metal Chelate) III, First Edition, ed. Takeichi Sakaguchi & Keihei Ueno," the chelate stability constants of these preferable chelators for calcium is 1.10 for maleic acid, 2.24 for malic acid, 2.49 for malonic acid, and 1.42 for lactic acid.

When the chelator is a salt of the organic acid, the salt may be a salt with an alkali metal such as sodium or potassium or an alkaline earth metal.

The chelator has a calcium-binding ability weaker than that of the anticoagulant in a blood sample or than that of the blood coagulation factor. Accordingly, the chelator does not interfere with the reaction between calcium and the anticoagulant or the blood coagulation reaction, and prevents excess calcium in the blood coagulation measurement system from binding to substances such as VLDL other than the blood coagulation factors in a blood sample, thereby enabling suppression of an optical change at an initial stage of the measurement.

The concentration of the chelator in the reagent for measuring blood coagulation can be suitably established depending on the type of the blood coagulation measurement method using the reagent for measuring blood coagulation and the type of the chelator. For example when the chelator is contained in the calcium solution used in the APTT measurement method, the concentration of the chelator in the calcium solution is preferably 20 to 120 mM, more preferably 40 to 100 mM.

The concentration of calcium in the calcium solution used in APTT measurement can be usually in the range of 20 to 25 mM. Accordingly, the concentration of the chelator in the calcium solution is set preferably 0.8- to 6-fold, more preferably 1.6-to 5-fold, relative to the concentration of calcium.

Preferably, the reagent for measuring blood coagulation further contain phospholipid.

The phospholipid is preferably that which is used as a component used in blood coagulation in a conventional reagent for measuring blood coagulation. A fatty acid side chain of the phospholipid is not particularly limited, but is preferably palmitic acid, oleic acid or stearic acid. The phospholipid includes phosphatidylserine, phosphatidylethanolamine, phosphatidylcholine, and the like. The phospholipid may be natural phospholipid derived from bovine brain, rabbit brain, human placenta, or soybean or may be prepared by genetic recombination.

The concentration of the phospholipid in the reagent for measuring blood coagulation can be suitably established depending on the type of the blood coagulation measurement method using the reagent for measuring blood coagulation. When the phospholipid is used in the APTT measurement method for example, the concentration of the phospholipid in the conventional APTT reagent can be 20 to 200 μg/mL. When the phospholipid is used in the PT measurement method, the concentration of the phospholipid in the conventional PT reagent can be 10 to 300 μg/mL.

Preferably, the reagent for measuring blood coagulation further contains calcium. Calcium is preferably in the form of a salt with an inorganic acid. Such calcium salt includes calcium chloride. Calcium salts other than salts of calcium with an inorganic acid include calcium lactate.

The concentration of calcium in the reagent for measuring blood coagulation can be suitably established depending on the type of the blood coagulation measurement method using the reagent for measuring blood coagulation. In the case of the APTT measurement method for example, the concentration of calcium in the calcium solution used in the conventional APTT measurement method can be about 20 to 25 mM. In the case of the PT measurement method, the concentration of calcium in the conventional PT reagent can be about 10 to 12.5 mM.

The pH of the reagent for measuring blood coagulation is preferably 6.0 to 8.0, more preferably 7.0 to 7.6. The pH of the reagent for measuring blood coagulation can be regulated with a buffer used in the conventional reagent for measuring blood coagulation. The buffer includes, for example, HEPES, TRIS etc.

Besides the components described above, the reagent for measuring blood coagulation may contain components in the conventionally known reagent for measuring blood coagulation in such a range that the effect of the present invention is not impaired. Such components include, for example, an activator, snake venom and a tissue factor. The activator includes ellagic acid, kaolin, celite, colloidal silica, silicic anhydride, alumina and magnesium. The snake venom includes Russell viper venom, tekistalin viper venom and ecarine viper venom. The tissue factor includes natural tissue thromboplastin derived from rabbit brain, human placenta or bovine brain, or genetically recombined tissue thromboplastin.

The reagent for measuring blood coagulation can be used as a reagent selected from the group consisting of a reagent for measuring prothrombin time (PT), a reagent for measuring activated partial thromboplastin time (APTT), a reagent for measuring a lupus anticoagulant (LA), a reagent for a thrombotest, a reagent for a hepaplastintest, and a reagent for quantifying a coagulation factor.

The present invention provides use of a reagent kit for measuring blood coagulation time, comprising a first reagent containing phospholipid and a second reagent containing calcium, wherein at least one of the first and second reagent comprises the calcium chelator described above.

More preferably, the reagent kit for measuring blood coagulation comprises the reagent for measuring blood coagulation as described above.

The reagent for measuring blood coagulation, when used for example in the APTT measurement method, maybe a reagent kit containing an APTT reagent, a calcium solution and a solution containing the chelator, may be a reagent kit containing an APTT reagent containing both the chelator and phospholipid, and a calcium solution, or may be a reagent kit containing an APTT reagent and a solution containing the chelator and calcium.

The reagent kit for measuring blood coagulation, when used for example in the PT measurement method, can be a reagent kit containing a PT reagent and the chelator.

The reagent kit for measuring blood coagulation can contain other reagents used as usual various reagents for measuring blood coagulation. The other reagents include standard plasma or standard serum, plasma made deficient in various blood coagulation factors, abuffersolution (for example, a veronal buffer solution for dilution of a blood sample or an imidazole buffer solution), and the like.

The present invention also provides a method for measuring blood coagulation time, comprising:

contacting a blood sample with calcium and phospholipid in the presence of a blood coagulation measurement reagent containing a calcium chelator interfering with the reaction between calcium and a substance other than the coagulation factor in the blood sample but not interfering with blood coagulation reaction, and measuring blood coagulation time.

The contacting step is carried out preferably in a measurement sample obtained by mixing the chelator-containing reagent for measuring blood coagulation according to the present invention, a blood sample, calcium and phospholipid.

The blood sample may be whole blood or plasma to which an anticoagulant is added. The plasma can be prepared from whole blood by a method known in the art. The anticoagulant may be any anticoagulant known in the art and is preferably sodium citrate. The mixing ratio of whole blood or plasma to the anticoagulant can be suitably selected depending on the type of blood coagulation time to be measured.

In the contacting step, the concentration of the chelator in the measurement sample containing a blood sample, the chelator, calcium and phospholipid can be suitably selected depending on the type of the blood coagulation measurement method, the type of the chelator, and the concentration of calcium in the measurement sample. In the case of the APTT measurement method for example, the concentration of the chelator in the measurement sample is preferably 7 to 40 mM, more preferably 13 to 33 mM. In the case of the PT measurement method for example, the concentration of the chelator in the measurement sample is preferably 3.5 to 20 mM, more preferably 6.5 to 16.5 mM.

In the contacting step, the concentration of calcium in the measurement sample is usually about 6.5 to 8.5 mM in the APTT measurement method for example, or is usually about 6.5 to 8.5 mM in the PT measurement method.

It follows that in the contacting step, the concentration of the chelator in the measurement sample is preferably set 0.8- to 6-fold, more preferably 1. 5- to 5-fold, relative to the concentration of calcium.

The concentration of phospholipid in the measurement sample is usually about 10 to 60 mM in the APTT measurement method for example, or is usually about 20 to 120 mM in the PT measurement method.

The contacting step can be conducted by mixing a blood sample, the chelator-containing reagent for measuring blood coagulation, a reagent containing calcium, and a reagent containing phospholipid.

In one embodiment, the reagent for measuring blood coagulation may further contain calcium.

In one embodiment, the reagent for measuring blood coagulation may further contain phospholipid.

In one embodiment, the reagent for measuring blood coagulation may further contain calcium and phospholipid.

The mixing ratio of the reagent for measuring blood coagulation to a blood sample can be suitably selected depending on the type of the blood coagulation time to be measured and according to the conventionally known method for measuring blood coagulation time.

In the APTT measurement method for example, a blood sample, the APTT reagent and the calcium solution can be mixed in a volume ratio of usually 1 : 1 : 1 to generate blood coagulation.

In the PT measurement method, a blood sample and the PT reagent can be mixed in a volume ratio of usually 1 : 2 to generate blood coagulation.

Preferably, the contacting step is conducted usually at a temperature of about 37°C. The contacting time can be suitably selected depending on the type of the blood coagulation measurement method.

The step of measuring blood coagulation time is conducted preferably by measuring optical information obtained from blood coagulation. The optical information includes a change in transmitted light, a change in scattered light, etc. The optical information can be detected by using a conventionally known blood coagulation measurement apparatus equipped with an optical information detection part. The blood coagulation measurement apparatus includes, for example, CS-2000i and CS-2100i, both of which are manufactured by Sysmex Corporation. These blood coagulation measurement apparatuses can measure a change in transmitted light, etc., to detect the process of blood coagulation in a measurement sample prepared in the contacting step.

EXPERIMENTS

Hereinafter, the present invention is described in more detail by reference to the Examples, but the present invention is not limited to the following examples.

<Reference Experiment 1>

In this reference experiment, it was confirmed that in the method for measuring a lupus anticoagulant (LA), an abnormal optical change in occurs at an initial stage of the measurement.

When a sample containing a large amount of a lipid component, such as chyle plasma, is used in measurement of blood coagulation, calcium is reacted with the lipid component in the plasma to cause agglutination, which may result in an abnormal optical change at an initial stage of the measurement. In this reference example, therefore, chyle plasma was used as a blood sample.

The measurement was conducted using a blood coagulation measurement apparatus CS-2000i (manufacturedby Sysmex Corporation) (Measurement Item: LA1). Specifically, 100 μL chyle plasma collected from a patient was incubated at 37°C for 4 minutes. 100 μL of an LA measurement reagent (LA1 Screening Reagent manufactured by Dade Behring) was added to the chyle plasma to prepare a measurement sample, and a change in transmitted light of the measurement sample was measured at 37°C for 4 minutes.

The results are shown in FIG. 2.

In FIG. 2, the value of transmitted light was rapidly reduced at an initial stage of the reaction for about 40 seconds just after the blood sample was mixed with the measurement reagent. From this result, it was confirmed that in the LAmeasurement method, an abnormal optical change is generated at an initial stage of the measurement.

<Reference Experiment 2>

In this reference experiment, it was confirmed that in the method for measuring prothrombin time (PT), an abnormal optical change occurs at an initial stage of the measurement.

The measurement was conducted using a blood coagulation measurement apparatus CS-2000i (manufacturedby Sysmex Corporation) (Measurement Item: PT). Specifically, 50 μL low-fibrinogen plasma collected from a patient was incubated at 37°C for 3 minutes. 100 μL of a PT measurement reagent (Thromborel S manufactured by Dade Behring) was added to the low-fibrinogen plasma to prepare a measurement sample, and a change in transmitted light of the measurement sample was measured at 37°C for 2 minutes.

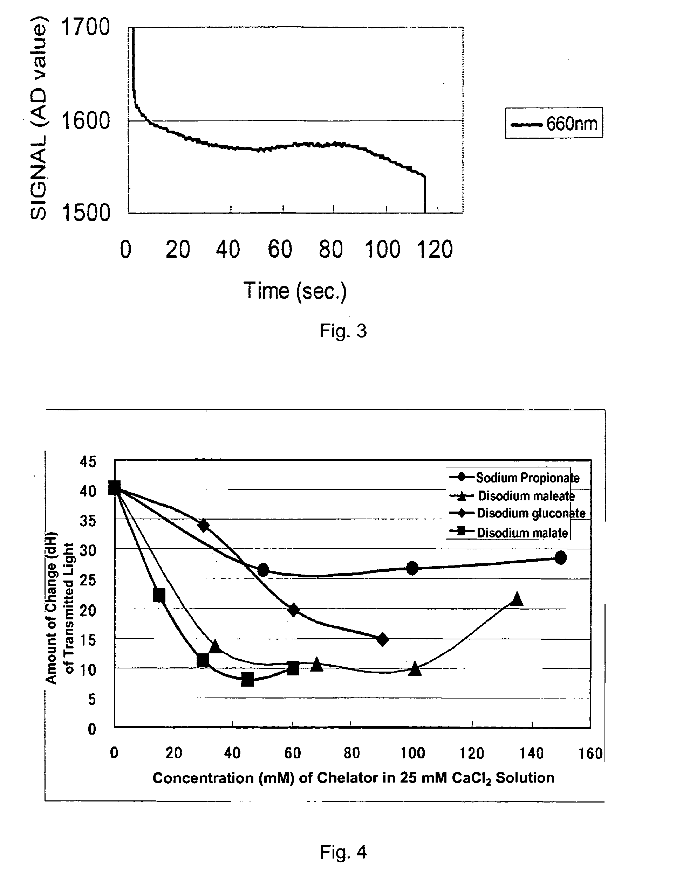

The results are shown in FIG. 3.

In this reference example, low-fibrinogen plasma is used as the blood sample. Accordingly, it is considered that in this measurement, an optical change due to blood coagulation very hardly occurs. In FIG. 3, however, the value of transmitted light was reduced at an initial stage of the reaction for about 40 seconds just after the blood sample was mixed with the measurement reagent, thus revealing that an abnormal optical change was generated at an initial stage of the measurement. The reason that such an abnormal optical change occurred in this reference example is not evident, and this abnormal optical change is considered attributable to the reaction between calcium in the reagent and a substance other than the blood coagulation factor in the plasma. The PT measurement reagent contains a relatively large amount of phospholipid.

Accordingly, it is considered that such an abnormal optical change is also attributable to the reaction between the phospholipid in the reagent and calcium.

<Experiment 1>

The reference examples showed that the abnormal optical change at an initial stage of the measurement can occur not only due to the reaction between lipid particles in a blood sample and calcium in the reagent, but also due to the reaction between phospholipid in the reagent and calcium. In this example, therefore, water was used in place of a blood sample to confirm that in the method for measuring activated partial thromboplastin time (APTT), an abnormal optical change occurs at an initial stage of the measurement. Then, whether the abnormal optical change at an initial stage of the measurement, generated by mixing the APTT reagent with calcium, can be suppressed with a chelator according to the present invention was examined.

The measurement was conducted using a blood coagulation measurement apparatus CS-2000i (manufacturedby Sysmex Corporation) (Measurement Item: APTT). Specifically, 5 μL of reverse osmosis membrane-treated water (RO water) in place of a blood sample was incubated at 37°C for 1 minute. 75 μL of an APTT measurement reagent (Actin FSL manufactured by Dade Behring) was added to the RO water and incubated at 37°C for 3 minutes. 75 μl of 25 mM CaCl$_2$ solution containing a chelator was added to the mixture to prepare a measurement sample, and a change in transmitted light of the measurement sample was measured at 37°C for 3 minutes. The type and concentration of the chelator in the CaCl$_2$ solution are as shown in Table 1 below. The measurement was carried out with the chelator four times at each concentration.

Table 1

| Chelator | Chelate Stability Constant for Ca | Concentration (mM) of Chelator in 25 mM CaCl$_2$ Solution | | | | |
|---|---|---|---|---|---|---|
| Sodium Propionate | 0.68 | 0 | 50 | 100 | 150 | |
| Disodium maleate | 1.1 | 0 | 24 | 68 | 101 | 135 |
| Disodium malate | 2.24 | 0 | 15 | 30 | 45 | 60 |
| Disodium gluconate | 1.21 | 0 | 30 | 60 | 90 | |

All the above chelators were purchased from Sigma Aldrich.

In each measurement, a change in transmitted light per minute for 3 minutes after the calcium chloride solution was added, was calculated using the following equation:

$$dH = (\text{[amount of transmitted light after a lapse of 3 minutes} - \text{[amount of transmitted light when CaCl}_2 \text{ solution was added]})/3$$

The value of dH reflects an optical change at an initial stage of the measurement, and it is estimated that as a smaller value of dH is indicative of a higher effect of suppressing the optical change at an initial stage of the measurement.

The results are shown in FIG. 4. FIG. 4 shows the average change in 4 measurements.

From FIG. 4, it could be confirmed that because the value of dH was 40 when the concentration of the chelator was 0 mM, the optical change at the initial stage can also occur by the reaction between phospholipid in the reagent and calcium.

In FIG. 4, it was found that the value of dH was reduced by incorporation of each chelator into the reagent. From this result, it was found that an abnormal optical change at the first stage of the measurement can be suppressed by using these chelators. In FIG. 4, the chelators, particularly maleic acid and malic acid, showed a high suppressing effect. For example, maleic acid showed the suppressing effect at a concentration of 20 to 120 mM and showed a higher suppressing effect at 30 to 100 mM. Malic acid showed the suppressing effect at a concentration of 20 mM or more and showed a higher suppressing effect at 30 to 60 mM. Gluconic acid showed the suppressing effect at a concentration of 40 mM or more and showed a higher suppressing effect at 60 to 90 mM. Propionic acid showed the suppressing effect at a concentration of 30 mM or more and showed a higher suppressing effect at 40 to 150 mM.

<Experiment 2>

Control plasma for commercial blood coagulation test was used to prepare pseudo-chyle plasma sample which were then used in measurement of APTT.

Intra Fat (manufactured by Takeda Pharmaceutical Company Limited) was added at a concentration of 0.2% to control plasma P for blood coagulation test (manufacturedby Dade Behring) to prepare pseudo-chyle plasma sample. The control plasma P is control plasma prepared such that its coagulation activity shows a low value by an abnormality in coagulation factors. Intra Fat is a lipid preparation. It is estimated that because the resulting pseudo-chyle plasma sample are low in coagulation activity, the optical change at an initial stage of the measurement, caused by the reaction between calcium in the reagent and the lipid component in the sample, can be easily detected.

The measurement was conducted in the same manner as in Example 1 except that the pseudo-chyle sample were used in an amount of 50 $\mu$l in place of RO water in Example 1, and the APTT reagent and 25 mM $CaCl_2$ solution to which the chelator had been added were used in an amount of 50 $\mu$l respectively, and the amount of change (dH) of transmitted light was calculated.

As the chelator, disodium malate added at a concentration of 0 or 30 mM to the $CaCl_2$ solution was used. The measurement was carried out with the chelator four times at each concentration.

The results are shown in Table 2 below. Table 2 shows the average change in 4 measurements.

Table 2

| | Concentration of Chelator in 25 mM $CaCl_2$ Solution | Amount of Change (dH) of Transmitted Light |
|---|---|---|
| Disodium malate | 0mM | 80 |
| | 30mM | 33 |

In Table 2, the value of dH was 80 when the concentration of malic acid as the chelator was 0 mM, and thus the optical change at an initial stage of the measurement, caused by the reaction between calcium in the reagent and the lipid component in the sample, could be confirmed.

In Table 2, it was found that the value of dH was reduced by incorporation of the chelator into the reagent. From this result, it was found that the abnormal optical change at an initial stage of the measurement, caused by the reaction between calcium in the reagent and the lipid component in the sample, can be suppressed by using the chelator.

<Experiment 3>

In this example, water was used in place of a blood sample to confirm that in the method for measuring thromboplastin time (PT), an abnormal optical change occurs at an initial stage of the measurement. Then, whether the abnormal optical change at an initial stage of the measurement, generated by phospholipid and calcium in the PT reagent, can be suppressed with a chelator according to the present invention was examined.

The measurement was conducted using a blood coagulation measurement apparatus CS-2000i (manufactured by Sysmex Corporation) (Measurement Item: PT). Specifically, 5 $\mu$L of reverse osmosis membrane-treated water (RO water) in place of a blood sample was incubated at 37°C for 3 minutes. 150 $\mu$L of a PT measurement reagent (Thromborel S manufactured by Dade Behring) containing disodium maleate as the chelator was added to the RO water to prepare a measurement sample, and a change in transmitted light of the measurement sample was measured at 37°C for 2 minutes. The concentration of disodium maleate in the PT measurement reagent is as shown in Table 3 below. The measurement was carried out with the chelator four times at each concentration.

The amount of change (dH) in transmitted light in each measurement was calculated. The results are shown in Table 3. Table 3 shows the average change in 4 measurements.

Table 3

| | Concentration of Chelator in 25 mM $CaCl_2$ Solution | Amount of Change (dH) of Transmitted Light |
|---|---|---|
| Disodium malate | 0mM | 29 |
| | 34mM | 13 |
| | 68mM | 8 |
| | 101mM | 9 |
| | 135mM | 6 |

From Table 3, it could be confirmed that because the value of dH was 29 when the concentration of maleic acid as the chelator was 0 mM, an optical change at an initial stage can also occur by the reaction between phospholipid and calcium in the reagent.

In Table 3, it was found that the value of dH was reduced by incorporation of the chelator into the reagent. From this result, it was found that an abnormal optical change at an initial stage of the measurement can be suppressed by using the chelator. In Table 3, maleic acid showed the suppressing effect at a concentration of 34 mM or more and showed a higher suppressing effect at 68 to 135 mM.

**Claims**

1. A method for measuring the coagulation time of a blood sample, comprising contacting the blood sample, calcium, and a compound selected from the group consisting of phospholipid, tissue factor, and a complex of tissue factor and phospholipid, in the presence of a calcium chelator,
   wherein the calcium chelator has a lower chelate stability constant for calcium than that of citric acid or its salt used generally as an anticoagulant and
   wherein the calcium chelator is chosen from the group consisting of maleic acid, malic acid, malonic acid, lactic acid, formic acid, acetic acid, butyric acid, isobutyric acid, valeric acid, isovaleric acid, succinic acid, fumaric acid, citraconic acid, itaconic acid, tricarballylic acid, propane-1,1,2,3-tetracarboxylic acid, butane-1,2,3,4- tetracarboxylic acid, glycolic acid, thioglycolic acid, tartaric acid, isocitric acid, pyruvic acid, oxaloacetic acid, and salts thereof, and measuring the coagulation time of the blood sample.

2. The method according to claim 1, wherein the blood sample is contacted with at least one activator selected from the group consisting of ellagic acid, kaolin, celite, colloidal silica, silicic anhydride, alumina and magnesium in the contacting step.

3. The method according to claims 1 or 2, wherein the calcium chelator is at least one selected from the group consisting of maleic acid, malic acid, and salts thereof.

4. The method according to claims 1 to 3, wherein the concentration of calcium chelator in a mixture of the calcium, phospholipid and blood sample is 0.8 to 6 times of the concentration of the calcium.

5. The method according to claims 1 to 4, wherein the coagulation time is measured by detecting an optical information of a mixture of the calcium, phospholipid and blood sample.

6. The method according to claims 1 to 4, wherein the coagulation time is measured by detecting a change of an optical information of a mixture of the calcium, phospholipid and blood sample.

7. The method according to claims 5 or 6, wherein the optical information is selected from the group consisting of transmitted light and scattered light.

8. The method according to any one of claims 1 to 7, wherein the method is for measurement of prothrombin time, measurement of activated partial thromboplastin time, measurement of lupus anticoagulant, thrombotest, hepaplastintest, or quantification of coagulation factor.

9. Use of a reagent comprising a calcium chelator in a method for measurement of the coagulation time of a blood sample, wherein said method comprises contacting the blood sample, calcium, and a compound selected from the group consisting of phospholipid, tissue factor, and a complex of tissue factor and phospholipid, in the presence of said calcium chelator, and measuring the coagulation time of the blood sample, and wherein said calcium chelator is chosen from the group consisting of maleic acid, malic acid, malonic acid, lactic acid, formic acid, acetic acid, butyric acid, isobutyric acid, valeric acid, isovaleric acid, succinic acid, fumaric acid, citraconic acid, itaconic acid, tricarballylic acid, propane-1,1,2,3-tetracarboxylic acid, butane-1,2,3,4- tetracarboxylic acid, glycolic acid, thioglycolic acid, tartaric acid, isocitric acid, pyruvic acid, oxaloacetic acid, and salts thereof.

10. Use of a reagent kit comprising a first and a second reagent, wherein the first reagent comprises at least one selected from the group consisting of phospholipid, tissue factor, and a complex of tissue factor and phospholipid, and the second reagent comprises calcium, wherein a calcium chelator is comprised in the first reagent, the second reagent or both, in a method for measurement of the coagulation time of a blood sample, wherein said method comprises contacting said blood sample, said first reagent, said second reagent and said calcium chelator, and measuring the coagulation time of the blood sample, and wherein said calcium chelator is chosen from the group consisting of maleic acid, malic acid, malonic acid, lactic acid, formic acid, acetic acid, butyric acid, isobutyric acid, valeric acid, isovaleric acid, succinic acid, fumaric acid, citraconic acid, itaconic acid, tricarballylic acid, propane-1,1,2,3-tetracarboxylic acid, butane-1,2,3,4-tetracarboxylic acid, glycolic acid, thioglycolic acid, tartaric acid, isocitric acid, pyruvic acid, oxaloacetic acid, and salts thereof.

11. Use according to claims 9 or 10, wherein the calcium chelator is at least one selected from the group consisting of maleic acid, malic acid, and salts thereof.

12. Use according to claims 9 to 11, wherein the concentration of calcium chelator in a mixture of the calcium, phospholipid and blood sample is 0.8 to 6 times of the concentration of the calcium.

13. Use according to claims 9 to 12, wherein the coagulation time is measured by detecting an optical information of a mixture of the calcium, phospholipid and blood sample.

14. Use according to claims 9 to 12, wherein the coagulation time is measured by detecting a change of an optical information of a mixture of the calcium, phospholipid and blood sample.

15. Use according to claims 13 or 14, wherein the optical information is selected from the group consisting of transmitted light and scattered light.

16. Use according to claims 9 to 15, wherein the method is for measurement of prothrombin time, measurement of activated partial thromboplastin time, measurement of lupus anticoagulant, thrombotest, hepaplastintest, or quantification of coagulation factor.

**Patentansprüche**

1. Verfahren zum Messen der Koagulationszeit einer Blutprobe, das das Inkontaktbringen der Blutprobe, Calcium und einer Verbindung, ausgewählt aus der Gruppe bestehend aus Phospholipid, Gewebefaktor und einem Komplex aus Gewebefaktor und Phospholipid, in der Gegenwart eines Calciumchelators umfasst, wobei der Calciumchelator eine niedrigere Chelatstabilitätskonstante für Calcium aufweist als Citronensäure oder deren Salze, die allgemein als Antikoagulans verwendet werden, und wobei der Calciumchelator ausgewählt ist aus der Gruppe bestehend aus Maleinsäure, Malinsäure, Malonsäure, Milchsäure, Ameisensäure, Essigsäure, Buttersäure, Isobuttersäure, Valeriansäure, Isovaleriansäure, Bernsteinsäure, Fumarsäure, Citraconsäure, Itaconsäure, Tricarballylsäure, Propan-1,1,2,3-tetracarbonsäure, Butan-1,2,3,4-tetracarbonsäure, Glycolsäure, Thioglycolsäure, Weinsäure, Isocitronensäure, Pyruvinsäure, Oxalessigsäure und Salzen davon, und Messen der Koagulationszeit der Blutprobe.

2. Verfahren nach Anspruch 1, wobei die Blutprobe mit mindestens einem Aktivator, ausgewählt aus der Gruppe bestehend aus Ellagsäure, Kaolin, Celite, kolloidaler Kieselerde, Siliciumdioxid, Aluminiumoxid und Magnesium, in dem Kontaktierungsschritt in Kontakt gebracht wird.

3. Verfahren nach den Ansprüchen 1 oder 2, wobei der Calciumchelator mindestens einer, ausgewählt aus der Gruppe bestehend aus Maleinsäure, Malinsäure und Salzen davon ist.

4. Verfahren nach den Ansprüchen 1 oder 3, wobei die Calciumchelatorkonzentration in einem Gemisch aus Calcium, Phospholipid und Blutprobe das 0,8- bis 6-flache der Calciumkonzentration beträgt.

5. Verfahren nach den Ansprüchen 1 bis 4, wobei die Koagulationszeit gemessen wird, indem eine optische Information eines Gemischs aus Calcium, Phospholipid und Blutprobe nachgewiesen wird.

6. Verfahren nach den Ansprüchen 1 bis 4, wobei die Koagulationszeit gemessen wird, indem eine Veränderung der optischen Information eines Gemischs aus Calcium, Phospholipid und Blutprobe nachgewiesen wird.

7. Verfahren nach Anspruch 5 oder 6, wobei die optische Information ausgewählt ist aus der Gruppe bestehend aus durchgelassenem Licht und gestreutem Licht.

8. Verfahren nach einem der Ansprüche 1 bis 7, wobei das Verfahren dem Messen der Prothrombinzeit, dem Messen der aktivierten partiellen Thromboplastinzeit, dem Messen des Lupusantikoagulans, Thrombotest, Hepaplastintest oder der Quantifizierung des Koagulationsfaktors dient.

9. Verwendung eines Reagens, das einen Calciumchelator umfasst, in einem Verfahren zum Messen der Koagulationszeit einer Blutprobe, wobei das Verfahren das Inkontaktbringen der Blutprobe, Calcium und einer Verbindung, ausgewählt aus der Gruppe, bestehend aus Phospholipid, Gewebefaktor und einen Komplex aus Gewebefaktor und Phospholipid in der Gegenwart des Calciumchelators und das Messen der Koagulationszeit der Blutprobe umfasst, und wobei der Calciumchelator ausgewählt ist aus der Gruppe bestehend aus Maleinsäure, Malinsäure, Malonsäure, Milchsäure, Ameisensäure, Essigsäure, Buttersäure, Isobuttersäure, Valeriansäure, Isovaleriansäure, Bernsteinsäure, Fumarsäure, Citraconsäure, Itaconsäure, Tricarballylsäure, Propan-1,1,2,3-tetracarbonsäure, Butan-1,2,3,4-tetracarbonsäure, Glycolsäure, Thioglycolsäure, Weinsäure, Isocitronensäure, Pyruvinsäure, Oxalessigsäure und Salzen davon.

10. Verwendung eines Reagenskits, der ein erstes und ein zweites Reagens umfasst, wobei das erste Reagens mindestens eines ausgewählt aus der Gruppe bestehend aus Phospholipid, Gewebefaktor und einem Komplex aus Gewebefaktor und Phospholipid umfasst und das zweite Reagens Calcium umfasst, wobei ein Calciumchelator in dem ersten Reagens, dem zweiten Reagens oder in beiden enthalten ist, in einem Verfahren zum Messen der Koagulationszeit einer Blutprobe, wobei das Verfahren das Inkontaktbringen der Blutprobe, des ersten Reagens, des zweiten Reagens und des Calciumchelators und das Messen der Koagulationszeit der Blutprobe umfasst, und wobei der Calciumchelator ausgewählt ist aus der Gruppe bestehend aus Maleinsäure, Malinsäure, Malonsäure, Milchsäure, Ameisensäure, Essigsäure, Buttersäure, Isobuttersäure, Valeriansäure, Isovaleriansäure, Bernsteinsäure, Fumarsäure, Citraconsäure, Itaconsäure, Tricarballylsäure, Propan-1,1,2,3-tetracarbonsäure, Butan-1,2,3,4-tetracarbonsäure, Glycolsäure, Thioglycolsäure, Weinsäure, Isocitronensäure, Pyruvinsäure, Oxalessigsäure und Salzen davon.

11. Verwendung nach den Ansprüchen 9 oder 10, wobei der Calciumchelator mindestens einer ausgewählt aus der Gruppe bestehend aus Maleinsäure, Malinsäure und Salzen davon ist.

12. Verwendung nach den Ansprüchen 9 bis 11, wobei die Calciumchelatorkonzentration in einem Gemisch aus Calcium, Phospholipid und Blutprobe das 0,8- bis 6fache der Calciumkonzentration beträgt.

13. Verwendung nach den Ansprüchen 9 bis 12, wobei die Koagulationszeit gemessen wird, indem eine optische Information eines Gemischs aus Calcium, Phospholipid und Blutprobe nachgewiesen wird.

14. Verwendung nach den Ansprüchen 9 bis 12, wobei die Koagulationszeit gemessen wird, indem eine Veränderung der optischen Information eines Gemischs aus Calcium, Phospholipid und Blutprobe nachgewiesen wird.

15. Verfahren nach den Ansprüchen 13 oder 14, wobei die optische Information ausgewählt ist aus der Gruppe bestehend aus durchgelassenem Licht und gestreutem Licht.

16. Verwendung nach den Ansprüchen 9 bis 15, wobei das Verfahren dem Messen der Prothrombinzeit, dem Messen der aktivierten partiellen Thromboplastinzeit, dem Messen des Lupusantikoagulans, Thrombotest, Hepaplastintest oder der Quantifizierung des Koagulationsfaktors dient.

**Revendications**

1. Procédé de mesure du temps de coagulation d'un échantillon de sang, qui comprend la mise en contact de l'échantillon de sang, de calcium et d'un composé choisi dans le groupe constitué d'un phospholipide, un facteur tissulaire et un complexe de facteur tissulaire et de phospholipide en présence d'un chélateur du calcium,
dans lequel le chélateur du calcium a une constante de stabilité de chélate pour le calcium inférieure à celle de l'acide citrique ou de son sel généralement utilisé en tant qu'anticoagulant et
dans lequel le chélateur du calcium est choisi dans le groupe constitué de l'acide maléique, l'acide malique, l'acide malonique, l'acide lactique, l'acide formique, l'acide acétique, l'acide butyrique, l'acide isobutyrique, l'acide valérique, l'acide isovalérique, l'acide succinique, l'acide fumarique, l'acide citraconique, l'acide itaconique, l'acide tricarballytique, l'acide propane-1,1,2,3-tétracarboxylique, l'acide butane-1,2,3,4-tétracarboxylique, l'acide glycolique, l'acide thioglycolique, l'acide tartrique, l'acide isocitrique, l'acide pyruvique, l'acide oxaloacétique et leurs sels, et la mesure du temps de coagulation de l'échantillon de sang.

2. Procédé selon la revendication 1, dans lequel l'échantillon de sang est mis en contact avec au moins un activateur choisi dans le groupe constitué de l'acide ellagique, du kaolin, de la célite, de la silice colloïdale, de l'anhydride silicique, de l'alumine et du magnésium dans l'étape de mise en contact.

3. Procédé selon les revendications 1 ou 2, dans lequel le chélateur du calcium est au moins un choisi dans le groupe constitué de l'acide maléique, l'acide malique, et leurs sels.

4. Procédé selon l'une quelconque des revendications 1 à 3, dans lequel la concentration en chélateur du calcium dans un mélange du calcium, du phospholipide et de l'échantillon de sang est de 0,8 à 6 fois la concentration du calcium.

5. Procédé selon les revendications 1 à 4, dans lequel le temps de coagulation est mesuré par détection des informations optiques d'un mélange du calcium, du phospholipide et de l'échantillon de sang.

6. Procédé selon les revendications 1 à 4, dans lequel le temps de coagulation est mesuré par détection d'une modification des informations optiques d'un mélange du calcium, du phospholipide et de l'échantillon de sang.

7. Procédé selon les revendications 5 ou 6, dans lequel les informations optiques sont choisies dans le groupe constitué de la lumière transmise et la lumière diffractée.

8. Procédé selon l'une quelconque des revendications 1 à 7, dans lequel le procédé est destiné à mesurer le temps de prothrombine, à mesurer le temps de thromboplastine partielle activée, à mesurer l'anticoagulant lupique, le thrombotest, l'hépaplastintest, ou la quantification du facteur de coagulation.

9. Utilisation d'un réactif comprenant un chélateur du calcium dans un procédé de mesure du temps de coagulation d'un échantillon de sang, dans laquelle ledit procédé comprend la mise en contact de l'échantillon de sang, du calcium et d'un composé choisi dans le groupe constitué d'un phospholipide, un facteur tissulaire et un complexe de facteur tissulaire et de phospholipide en présence dudit chélateur du calcium, et la mesure du temps de coagulation de l'échantillon de sang, et dans laquelle ledit chélateur du calcium est choisi dans le groupe constitué de l'acide maléique, l'acide malique, l'acide malonique, l'acide lactique, l'acide formique, l'acide acétique, l'acide butyrique, l'acide isobutyrique, l'acide valérique, l'acide isovalérique, l'acide succinique, l'acide fumarique, l'acide citraconique, l'acide itaconique, l'acide tricarballytique, l'acide propane-1,1,2,3-tétracarboxylique, l'acide butane-1,2,3,4-tétracarboxylique, l'acide glycolique, l'acide thioglycolique, l'acide tartrique, l'acide isocitrique, l'acide pyruvique, l'acide oxaloacétique et leurs sels.

10. Utilisation d'un kit de réactifs comprenant des premier et second réactifs, dans laquelle le premier réactif comprend au moins un réactif choisi dans le groupe constitué d'un phospholipide, un facteur tissulaire, et un complexe de facteur tissulaire et de phospholipide, et le second réactif comprend le calcium, dans laquelle un chélateur du calcium est compris dans le premier réactif, le second réactif, ou les deux, dans un procédé de mesure du temps de coagulation d'un échantillon de sang, dans laquelle ledit procédé comprend la mise en contact dudit échantillon de sang, dudit premier réactif, dudit second réactif et dudit chélateur du calcium, et la mesure du temps de coagulation de l'échantillon de sang, et dans laquelle ledit chélateur du calcium est choisi dans le groupe constitué de l'acide maléique, l'acide malique, l'acide malonique, l'acide lactique, l'acide formique, l'acide acétique, l'acide butyrique, l'acide isobutyrique, l'acide valérique, l'acide isovalérique, l'acide succinique, l'acide fumarique, l'acide citraconique, l'acide itaconique, l'acide tricarballytique, l'acide propane-1,1,2,3-tétracarboxylique, l'acide butane-1,2,3,4-tétracarboxylique, l'acide glycolique, l'acide thioglycolique, l'acide tartrique, l'acide isocitrique, l'acide pyruvique, l'acide oxaloacétique et leurs sels.

11. Utilisation selon les revendications 9 ou 10, dans laquelle le chélateur du calcium est au moins un choisi dans le groupe constitué de l'acide maléique, l'acide malique, et leurs sels.

12. Utilisation selon les revendications 9 à 11, dans laquelle la concentration en chélateur du calcium dans un mélange du calcium, du phospholipide et de l'échantillon de sang est de 0,8 à 6 fois la concentration du calcium.

13. Utilisation selon les revendications 9 à 12, dans laquelle le temps de coagulation est mesuré par détection d'informations optiques d'un mélange du calcium, du phospholipide et de l'échantillon de sang.

14. Utilisation selon les revendications 9 à 12, dans laquelle le temps de coagulation est mesuré par détection d'une modification des informations optiques d'un mélange du calcium, du phospholipide et de l'échantillon de sang.

**15.** Utilisation selon les revendications 13 ou 14, dans laquelle les informations optiques sont choisies dans le groupe constitué de la lumière transmise et de la lumière diffusée.

**16.** Utilisation selon les revendications 9 à 15, dans laquelle le procédé est destiné à mesurer le temps de prothrombine, à mesurer le temps de thromboplastine partielle activée, à mesurer l'anticoagulant lupique, le thrombotest, l'hépaplastintest, ou la quantification du facteur de coagulation.

**Contact with foreign matter**

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003138962 A

**Non-patent literature cited in the description**

- **TOH, CH et al.** *Blood,* 2000, vol. 100 (7), 2522-2529
- **TAKEICHI SAKAGUCHI ; KEIHEI UENO.** Kinzoku Chelate
- Kinzoku Chelate